# FASCICULE DE BREVET EUROPEEN

(11) **EP 2 307 404 B1**
(45) Date de publication et mention de la délivrance du brevet: **30.05.2018**
(21) Numéro de dépôt: 09772724.2
(22) Date de dépôt: 18.06.2009
(51) Int. Cl.: C07D 403/12, C07D 403/14, A61K 8/49, A61Q 5/12, C07D 239/47

(54) **COMPOSITION COSMÉTIQUE, COMPOSÉ ET PROCÉDÉ DE TRAITEMENT COSMÉTIQUE**
KOSMETISCHE ZUSAMMENSETZUNG, VERBINDUNG UND KOSMETISCHES BEHANDLUNGSVERFAHREN
COSMETIC COMPOSITION, COMPOUND, AND COSMETIC TREATMENT METHOD

(30) Priorité: 04.07.2008 FR 0854554; 14.07.2008 US 80285
(43) Date de publication de la demande: 13.04.2011
(73) Titulaire: L'Oréal, 75008 Paris (FR)
(72) Inventeur: MOUGIN, Nathalie, F-75011 Paris (FR); SCHULTZE, Xavier, F-93320 Les Pavillons Sous Bois (FR); DUBLANCHET, Anne-Claude, F-92160 Antony (FR); PHILIPPE, Michel, F-91320 Wissous (FR)
(74) Mandataire: Dodin, Catherine
(86) Numéro de dépôt international: PCT/FR2009/051166
(87) Numéro de publication internationale: WO 2010/001042

(56) Documents cités:
- WO-A-02/098377
- WO-A-03/032929
- WO-A-2008/059125
- JP-A- 2007 320 306
- VALÉRIE G. H. LAFITTE ETAL.: "Highly stable cyclic dimers based on non-covalent interactions" CHEMICAL COMMUNICATIONS - CHEMCOM., 2006, pages 2173-2175, XP002517554 GBROYAL SOCIETY OF CHEMISTRY.

## Description

La présente invention concerne un procédé de traitement cosmétique des matières kératiniques, notamment des cheveux, à l'aide d'une composition comprenant des composés susceptibles de former des liaisons hydrogène, ainsi que les compositions cosmétiques ainsi préparées.

En cosmétique, on cherche toujours à améliorer les propriétés des matières kératiniques et à lutter contre les dommages, tels que les agressions extérieures comme la pollution et le rayonnement ultraviolet ou les agressions chimiques comme celles provoquées par les traitements de coloration ou de permanente. Parmi les dégâts subis par le cheveu, on peut notamment citer la perte de brillance, l'augmentation du caractère hydrophile, la perte ou le décollement d'une partie des écailles, les difficultés de démêlage.
Pour améliorer les propriétés des cheveux, il est connu d'utiliser des compositions contenant des actifs cosmétiques pour apporter aux matières kératiniques telles que le cheveu tous les bienfaits liés à ces actifs cosmétiques. Toutefois, la rémanence et donc l'efficacité de ces actifs ne sont pas suffisantes, car ils peuvent être aisément éliminés au shampoing, ou encore ils ne forment pas un dépôt homogène à la surface du cheveu.

La présente invention a pour but de proposer une composition cosmétique susceptible d'être utilisée pour le traitement cosmétique des cheveux, et de leur apporter des propriétés cosmétiques de manière durable.
On a en effet constaté que des composés comportant des entités capables de former des interactions physiques entre elles pouvaient apporter des qualités cosmétiques intéressantes au cheveu. Ces composés sont notamment caractérisés par la présence d'au moins une entité capable de donner au moins 3 liaisons hydrogène, en particulier 4 liaisons hydrogène, ainsi que par leur faible masse.

La présente invention a donc pour objet un procédé de traitement cosmétique des matières kératiniques, consistant à appliquer sur lesdites matières kératiniques une composition cosmétique comprenant un composé de formule (I) : 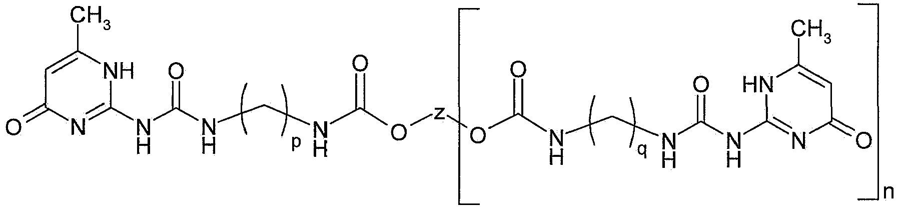 dans laquelle :
- n = 1
- p et q sont, indépendamment l'un de l'autre, des entiers compris entre 1 et 16;
- Z représente un radical multivalent :

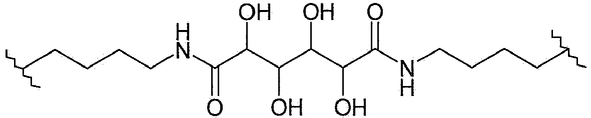

Un autre objet de l'invention est une composition cosmétique comprenant, dans un milieu cosmétiquement acceptable, au moins un tel composé de formule (I).
Les composés de formule (I) constituent encore un autre objet de l'invention.

On a constaté que le fait de déposer sur le cheveu ou de faire pénétrer dans le cheveu un composé selon l'invention permettait d'apporter un effet bénéfique au cheveu. Sans être tenu par la présente explication, on peut penser que les entités uréidopyrimidone sont capables de générer in situ dans ou sur le cheveu, un réseau réticulé, par associations physiques entre molécules. Déposé sur le cheveu ou la peau, ledit composé se trouve engagé et emprisonné dans un dépôt réticulé, ce qui accroît sa rémanence, en particulier au sébum, à l'eau et au shampooing. Par ailleurs, comme la réticulation est une réticulation physique, la rémanence de l'effet est possible tout en permettant le démaquillage du composé. L'élimination du dépôt peut consister notamment en un rinçage par une composition nettoyante appliquée à température ambiante ou à une température supérieure, ou par l'utilisation d'un démaquillant, ou en utilisant tout rupteur connu de liaison hydrogène.
La présente invention peut notamment permettre d'hydrater et de renforcer les matières kératiniques, et d'apporter de la douceur et de la brillance de façon durable de façon à ce que l'effet reste perceptible après au moins un shampooing. Par renforcement des matières kératiniques, on entend notamment une amélioration des propriétés mécaniques qui peut se traduire par :
- une augmentation de leur rigidité, ce qui leur procure davantage de résistance et de corps; ou bien
- une diminution de leur déformation en particulier en conditions humides, ce qui permet au cheveu de retrouver facilement sa forme initiale une fois séché et se traduit par une amélioration de la dynamique du cheveu; ou encore
- une meilleure résistance à des forces mécaniques de traction qui leur sont appliquées, par exemple lors d'un peignage, et qui peuvent entraîner la casse du cheveu.
Par ailleurs, le fait de pouvoir traiter le cheveu in situ peut également permettre d'apporter des propriétés de protection ou de réparation au cheveu, suite à un traitement de coloration, de décoloration, de permanente, de lissage ou de défrisage. Créer ce réseau réticulé dans le cheveu permet également d'éviter un dégorgement prématuré d'une teinture.
Les composés selon l'invention peuvent donc à la fois gainer le cheveu, et donc apporter notamment des propriétés de renforcement, et également pénétrer dans le cheveu et apporter in situ des propriétés de protection ou de réparation, notamment.

De nombreux composés incorporant des motifs ureïdopyrimidones (UPY) ont été décrits dans la littérature et étudiés pour leur propriété d'auto-assemblage par des laboratoires de recherche fondamentale. Toutefois aucun de ces documents ne décrit l'application de ces composés sur les matières kératiniques, ni même leur possible utilisation pour renforcer lesdites matières.
Dans le domaine cosmétique, on peut citer WO02098377 qui décrit d'une manière générale des composés à motifs UPY pour des applications cosmétiques sur la peau et les cheveux. On peut aussi citer WO2003032929 qui décrit la préparation de polymères supramoléculaires et leur utilisation dans des applications capillaires. On peut également citer WO2004016598 qui décrit la préparation de polymères supramoléculaires et leur utilisation dans des applications variées incluant les applications cosmétiques. Ou encore WO2005042641 qui décrit la préparation de polymères supramoléculaires, notamment de type polyuréthannes, et leur utilisation dans des applications variées incluant les applications cosmétiques. Mais aussi WO2008/059125 qui décrit, de façon générale, des composés colorants supramoléculaires à motifs UPY pour leur utilisation pour la coloration de matières kératiniques.
Dans l'ensemble de ces documents, les composés décrits sont des polymères, donc des composés de poids moléculaire élevé, qui ne vont donc pas pénétrer dans le cheveu pour lui apporter des propriétés bénéfiques, notamment en terme de protection du cheveu.

Le composé susceptible d'être employé dans le cadre de l'invention répond donc à la formule (I) : 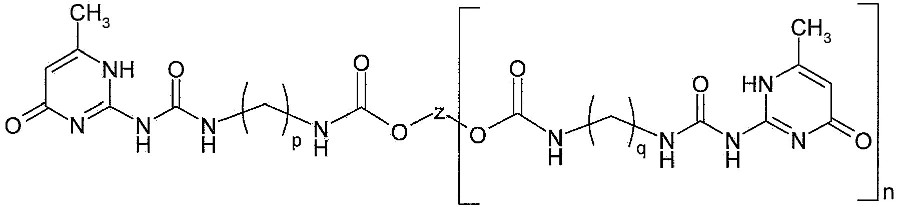 dans laquelle :
- n = 1
- p et q sont, indépendamment l'un de l'autre, des entiers compris entre 1 et 16, notamment entre 2 et 12, voire entre 3 et 8, et notamment 6;
- Z représente un radical multivalent :

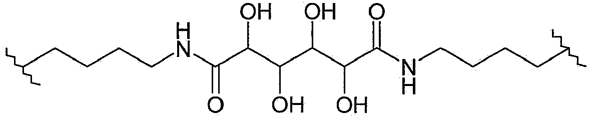

De préférence, le poids moléculaire en masse (Mw) du composé selon l'invention est inférieur ou égal à 1200 g/mol. Ce faible poids moléculaire favorise en particulier la pénétration des composés dans le cheveu.

Les composés de formule (I) peuvent être préparés selon toutes les méthodes connues.
Si l'on appelle A, le groupement suivant : 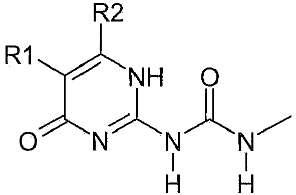 tel que R1 représente H et R2 représente CH₃.
et W le groupement suivant : 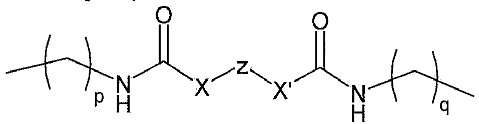 tel que X et X' représentent -O-.

les composés selon l'invention peuvent donc être schématisés de la manière suivante : A-W-(A)ₙ.
Ils peuvent être obtenus lors de la réaction :
- entre une fonction réactive liée au groupe A avec une fonction réactive portée par le groupement W; ou bien
- entre une fonction réactive liée à un précurseur du groupe A avec une fonction réactive portée par le groupement W pour former simultanément le groupe A et l'entité A-W;
les deux fonctions réactives étant bien entendu capables de réagir entre elles, et pouvant être liées directement ou par un segment divalent au groupe A et/ou au groupement W et/ou au précurseur dudit groupe A.

Les fonctions réactives peuvent de préférence être choisies parmi les fonctions :
- isocyanate -N=C=O ;
- isothiocyanate -N=C=S ;
- acide ou ester carboxylique -COORₐ ou ester activé COOR_{b}, avec Rₐ étant H ou un radical alkyle, linéaire ou ramifié en C₁-C₁₂, de préférence en C₁-C₄ et mieux un radical méthyle ou éthyle; et OR_{b} étant choisi parmi phenoxy, 4-nitrophenoxy, 2,4,5-trichlorophenoxy et les radicaux suivants : 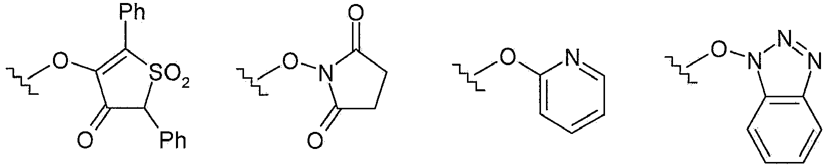
- halogénure d'acyle,
- acyl imidazole ou acyl benzotriazole de formule : 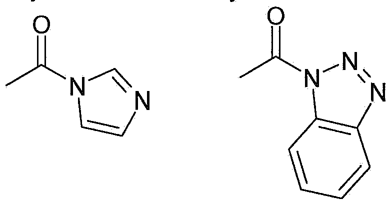
- anhydride d'acide,
- acide carbamique activé -NHCOX avec X= Cl, imidazole ou OR_{b} tel que défini ci-dessus;
- hydroxyle (OH) ou hydroxyle activé par exemple sous forme O-tosylate,
- amine primaire ou secondaire -N(Rₐ)₂, où Rₐ, identique ou différent, est tel que défini ci-dessus;
- une fonction choisie parmi : 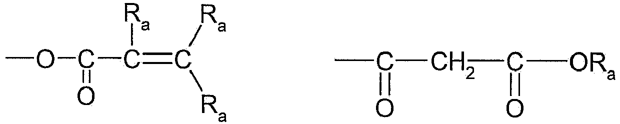
avec Rₐ, identique ou différent, est tel que défini ci-dessus.

De préférence, les fonctions réactives précurseur de la liaison entre W et A sont choisies parmi les fonctions isocyanate, amine, hydroxyle ou de formule :

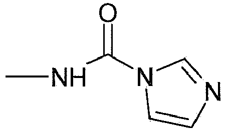

Une méthode d'obtention particulière de A-W- est celle décrite dans l'article de Katritzky et al., Comprehensive Organic Functional Group Transformations, Per-gamon :Oxford, 1995, vol 6, pp.500-506 ou bien dans Arkiv der Pharmazie, 314(1), 34-41, 1981.

On peut notamment faire réagir :
- une isocytosine B avec un acide carbamique activé : 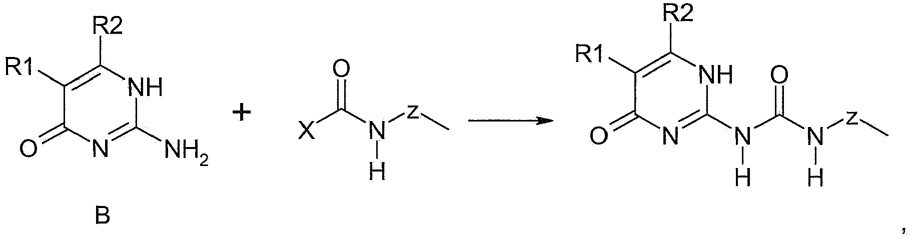
- une isocytosine B avec un isocyanate dérivé d'amine : 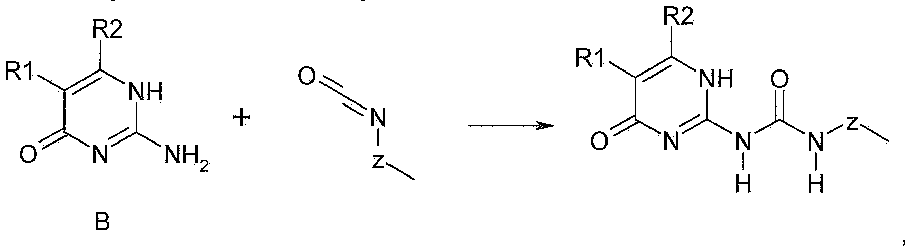
- une isocytosine à fonction carbamique activée C avec une aminé : 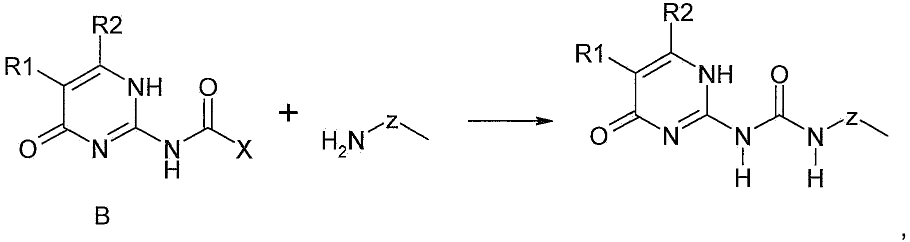
- un β-cétoester D avec un dérivé guanylalkylurée E : 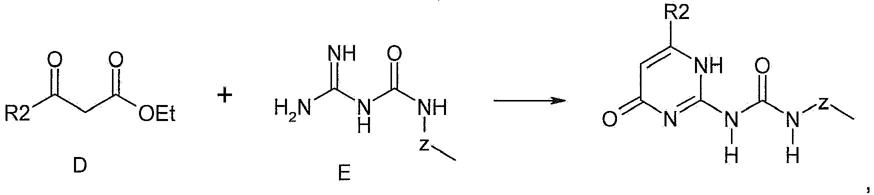
tel que R1 représente H et R2 représente CH₃.

Un autre procédé de préparation de A-W- consiste à synthétiser un composé du type : 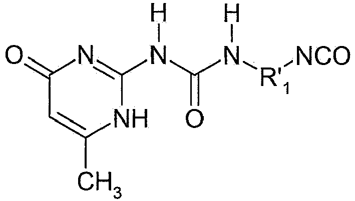 puis de le faire réagir sur un alcool ou une amine

Le groupement divalent R'1 représente par exemple un groupe choisi parmi: 1,2-éthylène; 1,6-hexylène; 1,4-butylène, 1,6-(2,4,4-triméthylhexylène); 1,4-(4-méthylpentylène), 1,5-(5-méthylhexylène), 1,6-(6-méthylheptylène); 1,5-(2,2,5-triméthylhexylène), 1,7-(3,7-diméthyloctylène); isophorone; 4,4'-méthylène-biscyclohexylène, tolylène,2-méthyl-1,3-phénylène; 4-méthyl-1,3-phénylène; 4,4-biphénylèneméthylène; et de préférence isophorone; -(CH₂)₂-; -(CH₂)₆-;-CH₂CH(CH₃)-CH₂-C(CH₃)₂-CH₂-CH₂; 4,4'-méthylènebiscyclohexylène;2-méthyl-1,3-phénylène. De préférence R'₁ = isophorone ou 1,6-hexylène.

De façon particulièrement préférée, les composés de formule (I) sont solubles ou dispersibles dans l'eau ou solubles dans de l'eau basifiée à l'aide d'une solution d'ammoniaque à 28% (le pH de la solution est alors compris entre 8 et 10).
Par soluble, on entend que le composé forme une solution limpide, à une concentration de 1% en poids dans le milieu, à 25°C, 1 atm.
Par dispersible, on entend que le composé forme dans le milieu, à concentration de 1% en poids à 25°C, 1 atm., une suspension ou dispersion stable de fines particules, généralement sphériques. Par stable, on entend que la suspension ne précipite pas et ne présente donc pas de dépôt visible. La taille moyenne des particules constituant la suspension ou la dispersion est de préférence inférieure à 1 µm et, plus généralement varie entre 5 et 400 nm, de préférence 10 à 250 nm. Ces tailles de particules sont mesurées par toute méthode classique de diffusion de lumière.

Les composés selon l'invention trouvent une application toute particulière dans le domaine de la cosmétique, notamment dans le domaine capillaire.
La quantité de composé présent dans les compositions cosmétiques de l'invention dépend bien entendu du type de composition et des propriétés recherchées, et peut varier à l'intérieur d'une gamme très large, comprise généralement entre 0,001% et 15% en poids, de préférence entre 0,005% et 10% en poids, notamment entre 0,01% et 8% en poids, voire entre 0,1 et 7% en poids, par rapport au poids total de la composition.

Les compositions cosmétiques peuvent bien évidemment comprendre un mélange de composés de formule (I).

Les compositions selon l'invention peuvent se présenter sous toutes les formes galéniques classiquement utilisées, et notamment sous forme d'une solution ou suspension aqueuse, alcoolique ou hydroalcoolique, ou huileuse; d'une solution ou d'une dispersion du type lotion ou sérum; d'une émulsion, notamment de consistance liquide ou semi-liquide, du type H/E, E/H ou multiple; d'une suspension ou émulsion de consistance molle de type crème (H/E) ou (E/H); d'un gel aqueux ou anhydre, ou de toute autre forme cosmétique. De préférence, la composition se présente sous forme de lotion, sérum ou suspension aqueuse.

Ces compositions peuvent être conditionnées, notamment dans des flacons pompes ou dans des récipients aérosols, afin d'assurer une application de la composition sous forme vaporisée (laque) ou sous forme de mousse. De telles formes de conditionnement sont indiquées, par exemple, lorsqu'on souhaite obtenir un spray ou une mousse, pour le traitement des cheveux. Dans ces cas, la composition comprend de préférence au moins un agent propulseur.

Les compositions selon l'invention comprennent un milieu cosmétiquement acceptable, c'est-à-dire un milieu compatible avec les matières kératiniques, notamment la peau du visage ou du corps, les lèvres, les cheveux, les cils, les sourcils et les ongles.

Ledit milieu comprend de préférence au moins un ingrédient cosmétique usuel, notamment choisi parmi les agents propulseurs; l'eau, les huiles carbonées; les huiles siliconées; les alcools en C8-C40, les esters en C8-C40, les acides en C8-C40; les alcools en C1-C7, les cétones, les solvants organiques, les tensioactifs non ioniques, les tensioactifs cationiques, les tensioactifs anioniques, les tensioactifs amphotères, les tensioactifs zwittérioniques; les filtres solaires; les agents hydratants; les agents antipelliculaires; les antioxydants; les agents réducteurs; les bases d'oxydation, les coupleurs, les agents oxydants, les colorants directs; les agents défrisants, les agents nacrants et opacifiants; les agents plastifiants ou de coalescence; les hydroxyacides; les pigments; les charges; les silicones; les épaississants, les émulsionnants; les polymères, l'urée, les agents de mise à pH basique. Ledit milieu peut bien évidemment comprendre plusieurs ingrédients cosmétiques figurant dans la liste ci-dessus.
Selon leur nature et la destination de la composition, les ingrédients cosmétiques usuels peuvent être présents en des quantités usuelles, aisément déterminables par l'homme du métier, et qui peuvent être comprises, pour chaque ingrédient, entre 0,01 à 80% en poids.

La composition peut notamment comprendre de l'eau, un ou plusieurs alcools en C1-C7, seul ou en mélange avec de l'eau, et notamment un mélange eau/éthanol, eau/isopropanol ou eau/alcool benzylique.
Les huiles carbonées, notamment hydrocarbonées, et/ou les huiles siliconées peuvent être présentes à raison de 0,01 à 20% en poids, notamment 0,02 à 10% en poids par rapport au poids total de la composition. On peut notamment citer les huiles végétales, animales ou minérales, hydrogénées ou non, les huiles synthétiques hydrocarbonées, cycliques ou aliphatiques, linéaires ou ramifiées, saturées ou insaturées, telles que par exemple les polyalpha-oléfines, en particulier les polydécènes et polyisobutènes; les huiles de silicone, volatiles ou non, organo-modifiées ou non, hydrosolubles ou non; les huiles fluorées ou perfluorées; leurs mélanges.
Les alcools, les esters et les acides, ayant 8 à 40 atomes de carbone, peuvent être présents à raison de 0,01 à 50% en poids, notamment 0,1 à 20% en poids par rapport au poids total de la composition.
On peut notamment citer les alcools gras à chaînes linéaires ou ramifiées en C12-C32, notamment en C12-C26, et en particulier l'alcool cétylique, l'alcool stéarylique, l'alcool cétylstéarylique, l'alcool isostéarylique, l'octyldodécanol, le 2-butyloctanol, le 2-hexyldécanol, le 2-undécylpentadécanol, l'alcool oléique ou l'alcool linoléique.
On peut également citer les alcools gras en C8-C40, notamment C16-C20, alcoxylés, notamment éthoxylés, de préférence comportant de 10 à 50 moles d'oxyde d'éthylène et/ou d'oxyde de propylène, tels que l'oleth-12, le ceteareth-12 et le ceteareth-20, l'alcool stéarylique oxypropyléné notamment comportant 15 moles d'oxyde de propylène, l'alcool laurylique oxyéthyléné, notamment comportant plus de 7 groupes oxyéthylénés, ainsi que leurs mélanges.
On peut citer également les acides gras à chaînes linéaires ou ramifiées en C16-C40, et notamment l'acide méthyl-18 eicosanoïque, les acides d'huile de coprah ou d'huile de coprah hydrogénée; l'acide stéarique, l'acide laurique, l'acide palmitique et l'acide oléique, l'acide béhénique, et leurs mélanges;
On peut encore citer les esters gras à chaînes linéaires ou ramifiées en C16-C40, tels que les esters de polyols dérivés d'acides gras comportant de 8 à 30 atomes de carbone, et leurs dérivés oxyalkylénés et notamment oxyéthylénés, les polyols étant préférentiellement choisis parmi les sucres, les C2-C6-alkylène glycols, le glycérol, les polyglycérols, le sorbitol, le sorbitan, les polyéthylèneglycols, les polypropylèneglycols et leurs mélanges.

Les tensioactifs, non ioniques, cationiques, anioniques, amphotères ou zwittérioniques, ainsi que leurs mélanges, peuvent être présents à raison de 0,01 à 50% en poids, notamment 0,05 à 40% en poids, voire 0,1 à 30% en poids, par rapport au poids total de la composition.
Les agents propulseurs peuvent être présents à raison de 5 à 90% en poids par rapport au poids total de la composition, et plus particulièrement à raison de 10 à 60% en poids.
Les filtres solaires peuvent être présents à raison de 0,01 à 20% en poids, notamment 0,5 à 10% en poids par rapport au poids total de la composition.
Les agents hydratants peuvent être présents à raison de 0,01 à 20% en poids, notamment 0,1 à 7% en poids par rapport au poids total de la composition.
Les agents antipelliculaires peuvent être présents à raison de 0,001 à 20% en poids, notamment 0,01 à 10% en poids par rapport au poids total de la composition, de préférence 0,1 à 5% en poids.
Les antioxydants peuvent être présents à raison de 0,05 à 1,5% en poids, par rapport au poids total de la composition.
Les agents réducteurs peuvent être présents à raison de 0,1 à 30% en poids, notamment 0,5 à 20% en poids par rapport au poids total de la composition.
Les bases d'oxydation peuvent être présentes en une quantité comprise entre 0,001 à 10% en poids, de préférence de 0,005 à 6% en poids, du poids total de la composition.
Les coupleurs peuvent être présents en une quantité comprise entre 0,001 et 10% en poids, de préférence de 0,005 à 6% en poids, du poids total de la composition. Les agents oxydants peuvent être présents en une quantité comprise entre 1 et 40% en poids, de préférence entre 1 et 20% en poids, par rapport au poids de la composition.
Les colorants directs peuvent être présents en une quantité comprise entre 0,001 à 20% en poids, de préférence de 0,01 à 10% en poids, par rapport au poids total de la composition.
Les agents défrisants peuvent être présents à raison de 0,01 à 3,5% en poids, notamment 0,05 à 1,5% en poids par rapport au poids total de la composition. Les agents nacrants et opacifiants peuvent être présents à raison de 0,01 à 3% en poids, notamment 0,05 à 2,5% en poids par rapport au poids total de la composition.
Les agents plastifiants ou de coalescence peuvent être présents à raison de 0,1 à 25% en poids, notamment 1 à 10% en poids par rapport au poids total de la composition.
Les hydroxyacides peuvent être présents à raison de 1 à 10% en poids, notamment 2 à 5% en poids par rapport au poids total de la composition.
Les pigments et charges peuvent être présents à raison de 0,01 à 50% en poids, notamment 0,02 à 30% en poids par rapport au poids total de la composition.
Les silicones peuvent être volatiles ou non; on peut notamment citer les polyorganosiloxanes, modifiés ou non, à savoir les huiles, les gommes et les résines de polyorganosiloxanes, telles quelles ou sous forme de solutions dans des solvants organiques, ou sous forme d'émulsions ou de microémulsions. Elles peuvent être présentes en une quantité de 0,01 à 40% en poids, notamment 0,05 à 20% en poids, par rapport au poids total de composition.
Les épaississants peuvent être présents à raison de 0,01 à 10% en poids, notamment de 0,1 à 5% en poids par rapport au poids total de la composition.
Les polymères, notamment hydrosolubles ou solubles dans les huiles carbonées et/ou siliconées, peuvent être présents à raison de 0,01 à 20% en poids, notamment 0,1 à 10% en poids par rapport au poids total de la composition.
Parmi les agents de mise à pH basique, on peut citer l'ammoniaque ainsi que les bases couramment utilisées en cosmétique.
L'homme de métier veillera à choisir les ingrédients entrant dans la composition, ainsi que leurs quantités, de manière à ce qu'ils ne nuisent pas aux propriétés des compositions de la présente invention.

La composition cosmétique selon l'invention peut se présenter sous la forme d'un produit de soin, de nettoyage et/ou de maquillage de la peau du corps ou du visage, des lèvres, des sourcils, des cils, des ongles et des cheveux, d'un produit solaire ou autobronzant, d'un produit d'hygiène corporelle, d'un produit capillaire, notamment de soin, de nettoyage, de coiffage, de mise en forme, de coloration des cheveux.

Elle trouve notamment une application particulièrement intéressante dans le domaine capillaire, notamment pour le maintien de la coiffure ou la mise en forme des cheveux, ou encore le soin, le traitement cosmétique ou le nettoyage des cheveux. Les compositions capillaires sont de préférence des shampooings, des après-shampooings, des gels de coiffage ou de soin, des lotions ou crèmes de soin, des conditionneurs, des lotions de mise en plis, des lotions pour le brushing, des compositions de fixation et de coiffage telles que les laques ou spray; de lotion restructurante pour cheveux; de lotion ou gel antichute, de shampoing antiparasitaire, de lotion ou shampoing antipelliculaire, de shampoing traitant antiséborrhéique. Elle peut notamment se présenter sous la forme d'un produit de coloration capillaire, notamment coloration d'oxydation, éventuellement sous forme de shampoing colorant; sous forme de composition de permanente, de défrisage ou de décoloration, ou encore sous forme de composition à rincer ou non, à appliquer avant ou après une coloration, une décoloration, une permanente ou un défrisage ou encore entre les deux étapes d'une permanente ou d'un défrisage.

La composition selon l'invention peut également se présenter sous la forme d'une composition de soin, notamment hydratant, pour la peau du corps ou du visage, les lèvres et/ou les phanères, notamment d'un produit de soin destiné à traiter cosmétiquement la peau et notamment à l'hydrater, la lisser, la dépigmenter, la nourrir, la protéger des rayons solaires, ou lui conférer un traitement cosmétique spécifique. Ainsi elle peut être une base de soin pour les lèvres, une base fixante pour rouge à lèvres, une composition de protection solaire ou de bronzage artificiel, une composition de soin (de jour, de nuit, anti-âge, hydratante) pour le visage; une composition matifiante; une composition de nettoyage de la peau, par exemple un produit démaquillant ou un gel pour le bain ou la douche, ou un pain ou savon de nettoyage; une composition d'hygiène corporelle notamment un produit déodorant, anti-transpirant, ou encore une composition dépilatoire, un gel ou lotion après-rasage. Elle peut encore se présenter sous la forme d'un produit de maquillage de la peau du corps ou du visage, des lèvres, des cils, des ongles ou des cheveux; en particulier un fond de teint, un blush, un fard à joues ou à paupières, un produit anti-cernes, un eye-liner, un mascara, un rouge à lèvres, un brillant à lèvres, un crayon à lèvres; un vernis à ongles, un soin des ongles; un produit de tatouage temporaire de la peau du corps.

De manière encore plus particulière, la composition selon l'invention trouve une application intéressante pour le soin et le traitement cosmétique, notamment la protection, des cheveux, en particulier des cheveux affaiblis et/ou abîmés, par exemple par des traitements chimiques ou mécaniques; on peut notamment utiliser les composés selon l'invention en post-traitement, après une étape de coloration, de décoloration ou de défrisage des cheveux.

L'invention a donc pour objet un procédé de traitement cosmétique, notamment de maquillage, de soin, de nettoyage, de coloration, de mise en forme, des matières kératiniques, notamment de la peau du corps ou du visage, des lèvres, des ongles, des cheveux et/ou des cils, comprenant l'application sur lesdites matières d'une composition cosmétique comprenant au moins un composé selon l'invention.

De préférence, il s'agit d'un procédé de traitement cosmétique pour le conditionnement des cheveux, en particulier pour leur apporter du corps et/ou de la nervosité, ou améliorer le démêlage, le lissage, la peignabilité, la réparation et la maniabilité de la chevelure. Il peut s'agir d'un procédé de réparation et/ou de protection des cheveux abîmés ou affaiblis.

Dans un mode de réalisation particulier du procédé de traitement, on peut appliquer la composition selon l'invention en une fois ou, de préférence, en plusieurs fois (multi-application).
Ainsi, on peut appliquer une composition comprenant les composés selon l'invention, en une quantité par exemple de 0,05 à 1%, notamment 0,1 à 0,5% en poids, une première fois sur les cheveux, laisser reposer pendant 2 à 20, notamment 10 minutes, éventuellement en chauffant à une température inférieure à 65°C, ou bien au fer à lisser ou à friser, par exemple pendant quelques secondes, puis éventuellement sécher les cheveux avant d'appliquer une seconde fois la composition, et à nouveau laisser reposer pendant 2 à 20, notamment 10 minutes, avant d'éventuellement chauffer à une température inférieure à 65°C, ou bien au fer à lisser ou à friser, par exemple pendant quelques secondes, puis sécher ou laisser sécher. Il est possible d'effectuer une troisième application de la composition. Cette application en plusieurs fois de la composition peut notamment permettre d'améliorer la pénétration des composés à l'intérieur du cheveu, et donc améliorer la réparation du cheveu in situ.

Dans un autre mode de réalisation du procédé selon l'invention, il est possible d'appliquer sur les cheveux une composition comprenant les composés selon l'invention, en une quantité par exemple de 0,05 à 15%, notamment 0,5 à 10% en poids, de préférence 1 à 8% en poids, laisser reposer pendant 15 à 45, notamment 30 minutes, de préférence en chauffant à une température inférieure à 65°C, ou bien au fer à lisser ou à friser, par exemple pendant quelques secondes, puis rincer et laisser sécher ou sécher.

L'invention est illustrée plus en détail dans les exemples de réalisation suivants.

### Exemple 1

### Préparation du (5R,6S,7R,8S)-5,6,7,8-tetrahydroxy-23-[(6-méthyl-4-oxo-1,4-dihydropyrimidin-2-yl)amino]-4,9,14,23-tetraoxo-13-oxa-3,10,15,22-tetraazatricos-1-yl[6-({[(6-méthyl-4-oxo-1,4-dihydropyrimidin-2-yl)amino]carbonyl}amino)hexyl] carbamate

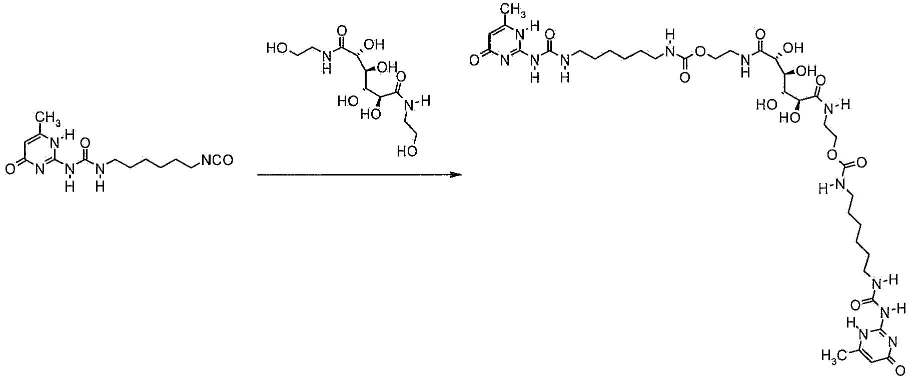

On ajoute à une solution de 0,5 g de (2R,3S,4R,5S)-2,3,4,5-tetrahydroxy-N,N'-bis(2-hydroxyéthyl)hexanediamide (1,7 mmol) et 0,5 ml de dibutyl dilaurate étain dans 50 ml de 2-méthyl-tetrahydrofurane, 1 g de 1-(6-isocyanatohexyl)-3-(6-méthyl-4-oxo-1,4-dihydropyrimidin-2-yl)urée (3,4 mmol) préalablement préparé selon la procédure décrite par Meijer et al. La solution est agitée à reflux pendant 48 heures. On ajoute 20 ml d'éthanol et on filtre le produit final, qui est séché sous pression réduite.
On obtient 1 g (1,1 mmol) de composé sous forme de poudre blanche.
¹H RMN (DMSO): δ 1.22-1.39 ppm (m, 8H); 1.40-1.63 ppm (m,8H); 2.05-2.18 ppm (s, 6H); 3.08-3.2 pmm (m,8H); 3.29-3.38 ppm (m, 8H), 3.39-3.47 ppm (q, 1H), 3.75-3.89 ppm (m, 1H), 4.07-4.19 ppm (d, 1H), 4.3-4.45 ppm (m, 1H), 4.61-4.75 ppm (t, 1H), 5.18-5.28 ppm (d, 1H), 5.74-5.86 ppm (s, 2H) (conforme à la structure attendue).

### Exemple 2

On mélange 0,4 g de composé de l'exemple 1 dans 4 ml d'eau, et on ajoute 1,3 ml d'une solution d'ammoniac à 32%. Le mélange est agité pendant 10 minutes à 25°C. On obtient une composition que l'on peut appliquer sur les cheveux.
Ainsi, on peut immerger pendant 30 minutes à 65°C des mèches de cheveux de type SA20 dans cette composition. On peut ensuite essorer et peigner la mèche, puis la rincer et la sécher à l'étuve à 60°C pendant 30 minutes.

## Revendications

1. Composition cosmétique comprenant, dans un milieu cosmétiquement acceptable, au moins un composé de formule (I) :

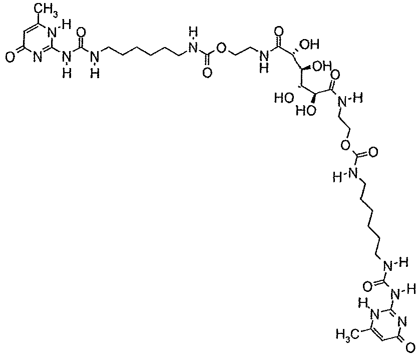

2. Composition selon l'une des revendications précédentes, dans laquelle le composé de formule (I) est présent à raison de 0,001% et 15% en poids, de préférence entre 0,005% et 10% en poids, notamment entre 0,01% et 8% en poids, voire entre 0,1 et 7% en poids, par rapport au poids total de la composition.

3. Composition selon l'une des revendications précédentes, se présentant sous la forme d'une composition capillaire, notamment de soin, de nettoyage, de coiffage, de maintien, de mise en forme, de coloration des cheveux.

4. Procédé de traitement cosmétique des matières kératiniques, consistant à appliquer sur lesdites matières kératiniques une composition cosmétique telle que définie à l'une des revendications 1 à 3.

5. Procédé selon la revendication 4, dans lequel les matières kératiniques sont les cheveux.

6. Procédé selon l'une des revendications 4 à 5, dans lequel il s'agit d'un procédé de traitement cosmétique pour le conditionnement des cheveux, en particulier pour leur apporter du corps et/ou de la nervosité, ou améliorer le démêlage, le lissage, la peignabilité, la réparation et la maniabilité de la chevelure.

7. Procédé selon l'une des revendications 4 à 6, dans lequel il s'agit d'un procédé de réparation et/ou de protection des cheveux abîmés ou affaiblis.

8. Composé de formule :

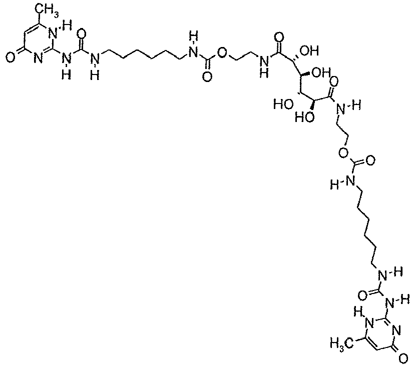

## Patentansprüche

1. Kosmetische Zusammensetzung, die in einem kosmetisch unbedenklichen Medium mindestens eine Verbindung der Formel (I): 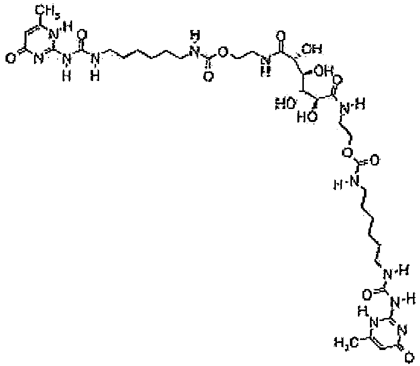 umfasst.

2. Zusammensetzung nach einem der vorhergehenden Ansprüche, wobei die Verbindung der Formel (I) in einer Menge von 0,001 und 15 Gew.-%, vorzugsweise zwischen 0,005 und 10 Gew.-%, insbesondere zwischen 0,01 und 8 Gew.-% oder sogar zwischen 0,1 und 7 Gew.-%, bezogen auf das Gesamtgewicht der Zusammensetzung, vorliegt.

3. Zusammensetzung nach einem der vorhergehenden Ansprüche, die in Form einer Haarzusammensetzung, insbesondere einer Haarpflege-, Haarreinigungs-, Haarfrisier-, Haarhalt-, Haarformungs-, Haarfärbezusammensetzung, vorliegt.

4. Verfahren zur kosmetischen Behandlung von Keratinmaterialien, bei dem man auf die Keratinmaterialien eine kosmetische Zusammensetzung gemäß einem der Ansprüche 1 bis 3 aufbringt.

5. Verfahren nach Anspruch 4, bei dem es sich bei den Keratinmaterialien um die Haare handelt.

6. Verfahren nach einem der Ansprüche 4 bis 5, bei dem es sich um ein kosmetisches Behandlungsverfahren zur Konditionierung der Haare handelt, insbesondere um diesen Fülle und/oder Sprungkraft zu verleihen oder die Entwirrbarkeit, Glättung, Kämmbarkeit, Reparatur und Handhabbarkeit der Kopfhaare zu verbessern.

7. Verfahren nach einem der Ansprüche 4 bis 6, bei dem es sich um ein Verfahren zur Reparatur und/oder zum Schutz von geschädigten oder geschwächten Haaren handelt.

8. Verbindung der Formel:

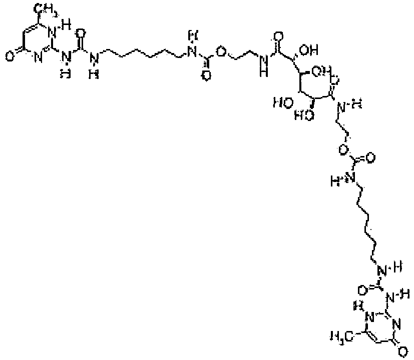

## Claims

1. Cosmetic composition comprising, in a cosmetically acceptable medium, at least one compound of formula (I) :

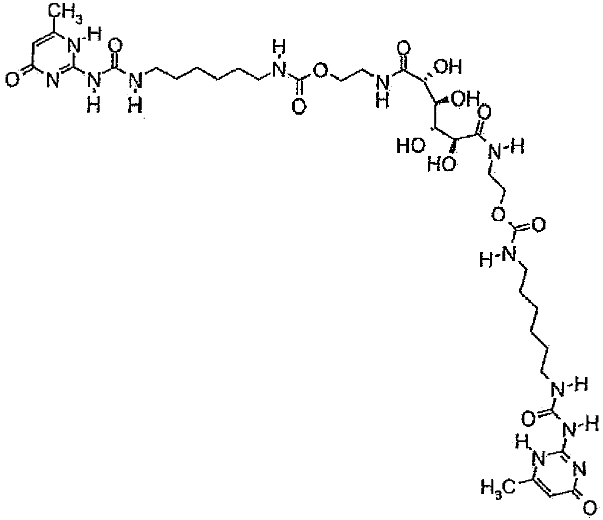

2. Composition according to one of the preceding claims, in which the compound of formula (I) is present in a proportion of from 0.001% and 15% by weight, preferably between 0.005% and 10% by weight, in particular between 0.01% and 8% by weight, or even between 0.1% and 7% by weight, relative to the total weight of the composition.

3. Composition according to either of the preceding claims, which is in the form of a hair composition, in particular a hair care, cleansing, styling, form retaining, shaping or dyeing composition.

4. Method for cosmetically treating keratin materials, which comprises applying a cosmetic composition as defined in one of Claims 1 to 3 to said keratin materials.

5. Method according to Claim 4, in which the keratin materials are the hair.

6. Method according to either of Claims 4 and 5, in which it is a cosmetic treatment method for conditioning the hair, in particular for giving it body and/or liveliness, or improving disentangling, smoothing, combability, repair and manageability of the head of hair.

7. Method according to one of Claims 4 to 6, in which it is a method for repairing and/or protecting damaged or weakened hair.

8. Compound of formula:
